Europäisches Patentamt

**European Patent Office**

Office européen des brevets

(19)

(11) Publication number: **0 113 194**
**A2**

(12)     # EUROPEAN PATENT APPLICATION

(21) Application number: **83307314.1**

(22) Date of filing: **01.12.83**

(51) Int. Cl.³: **G 01 N 33/22**
**G 01 T 1/38**

(30) Priority: **02.12.82 US 446095**

(43) Date of publication of application:
**11.07.84 Bulletin 84/28**

(84) Designated Contracting States:
**AT BE CH DE FR GB IT LI LU NL SE**

(71) Applicant: **USS ENGINEERS AND CONSULTANTS, INC.**
**600 Grant Street**
**Pittsburgh Pennsylvania 15230(US)**

(72) Inventor: **Huffman, Gerald Page**
**164 Jamison Lane**
**Monroeville Pennsylvania 15146(US)**

(72) Inventor: **Huggins, Frank Edward**
**3396 Douglas Drive**
**Murrysville Pennsylvania 15668(US)**

(74) Representative: **Lerwill, John et al,**
**A.A. Thornton & Co. Northumberland House 303-306**
**High Holborn**
**London, WC1V 7LE(GB)**

(54) **Method of determining iron, pyrite, or ash contents of coal.**

(57) A method for determining iron or pyrite or ash content of coal comprises measuring the attenuation or scattering by a coal sample of 14 keV gamma rays from a ⁵⁷Co Mossbauer source in a non-magnetic metallic matrix, both with the source at rest or vibrating with a maximum speed of less than 1mm/sec and also with the source vibrating with a peak speed of at least 5 mm/sec, and generating respective output signals as a function of said attenuation or scattering.

EP 0 113 194 A2

# METHOD OF DETERMINING IRON, PYRITE, OR ASH CONTENTS OF COAL

The present invention relates to the determination of iron, pyrite or ash content of coal.

Because of the deleterious effects of sulfur and ash in coal-utilization technologies, coals rich in these constituents are usually cleaned by coal-preparation procedures prior to use. Such procedures will usually remove sulfur in the form of pyrite ($FeS_2$), and up to 80 percent of the total mineral matter or ash in the as-mined coal. Coal-cleaning procedures could be made more efficient if there were a more rapid feedback for process control of the ash and total or pyritic sulfur contents of product coal. In current practice, there is typically a delay of several hours between sampling of the product and reporting of the analytical results. This delay could result in many thousands of tons of coal being outside required specifications before a problem was recognized.

It has been demonstrated that Mossbauer spectroscopy can provide an accurate, but relatively slow, determination of the pyrite content of coal, see Huffman and Huggins, Fuel 1978 vs. 57, p. 592, October 1978. Subsequently, Jaggi and Rao proposed that the resonant absorption by coal of 14 keV gamma rays

emitted by $^{57}$Fe nuclei in a special radioactive Mossbauer source prepared from pyrite could be used as the basis for a rapid measurement of pyrite sulfur, see Fuel vs. 58,. pp. 688-689, September 1979. To our knowledge, a Mossbauer pyritemeter has never been demonstrated experimentally.

We have determined that a pyritemeter modified from that proposed by Jaggi and Rao can be made to provide an estimate of pyrite in coal in about a minute, and that combinations of the pyritemeter with a Mossbauer gamma-ray attenuation measurement or certain X-ray-based ash monitors can provide refined methods for analyzing for the ash content of coal. Since the combined units are fast and can be continuous, they can be used in coal blending and monitoring systems using large quantities of coal, such as in coke plants and wherever coal is used as a fuel.

According to the present invention, there is provided a method of determing iron, pyrite or ash content of coal, comprising measuring the attenuation or scattering by a coal sample of 14 kV gamma rays from a Mossbauer source of $^{57}$Co in a non-magnetic metallic matrix, the measuring being performed both with the source at rest or vibrating with a peak speed of less than 1mm/sec and also with the source vibrating with a peak speed of at least 5mm/sec, and generating

- 3 -

respective output signals representing said attenuation or scattering.

The invention is further described, by way of example, with reference to the accompanying drawings, in which:

Figure 1 shows transmission count rates, as a function of vibration amplitude for a pyrite-rich coal, using a $^{57}Co(Pd)$ source.

Figures 2 and 3 show the effects on gamma-ray attenuation (transmission) and Mossbauer absorption caused by experimental additions of pyrite and ash as represented by $SiO_2$.

Figure 4 is a more or less diagrammatic representation of our preferred apparatus.

Figure 5 illustrates the relationships obtained with our apparatus.

Figure 6 illustrates the effects of introducing factors relating to sources of iron other than pyrite.

Figure 7 is a reproduction of certain relevant Mossbauer absorption peaks (spectra).

Figure 8 correlates % ash in coal to ln $(I_o/I_m)$. The light lines connect raw and washed coals from the same mine.

Figures 9a and 9b compare measured and calculated ratios of the absorption data.

- 4 -

Figure 10 is a graphical illustration of an application of equation (19).

Figure 11 illustrates the suggested use of our variation of the invention for measuring weights of coals.

In Figure 12 a further more or less diagrammatic illustration of our invention is presented showing the transmissions and scattering of radiation.

Jaggi and Rao proposed using $^{57}$Co-doped pyrite as a source so that when at rest the gamma rays emitted without recoil by this source would be of exactly the right energy for recoilless resonant absorption by pyrite in the sample. The additional absorption by the sample due to this nuclear process would be proportional to the pyrite content of the coal. This resonance between source and sample can be easily eliminated by rapidly vibrating the source and altering the energy of the source gamma rays by means of the Doppler effect.

Unfortunately, to our knowledge a usable $^{57}$Co-doped pyrite source has never been prepared and so this concept has not been tested experimentally. To circumvent this problem, we devised a modification to the experiment in which a standard Mossbauer source was used.

- 5 -

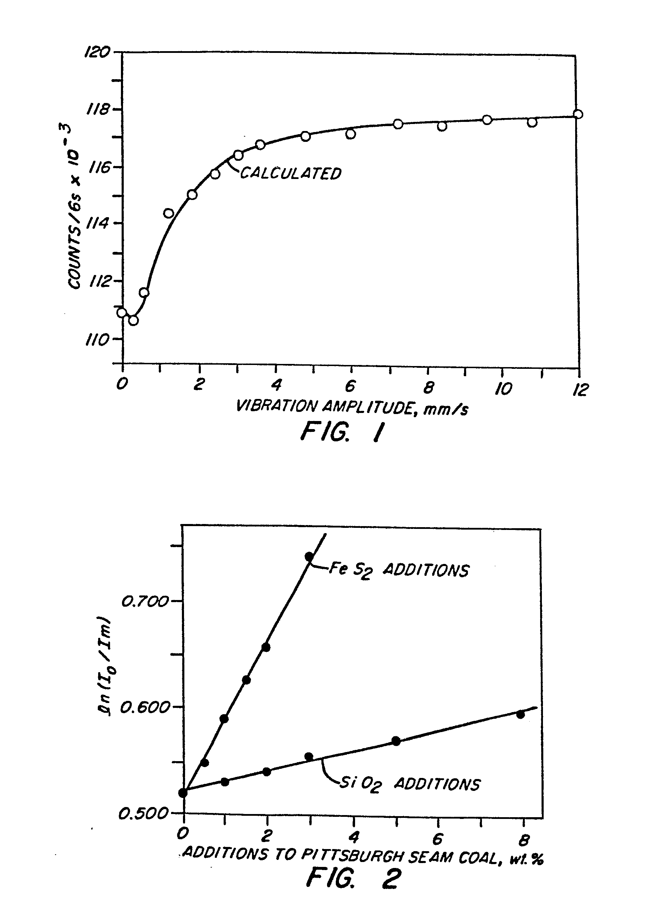

The standard $^{57}$Co-doped palladium source used for $^{57}$Fe Mossbauer experiments emits a single line at energy of 0.18 mm/s relative to iron metal, which overlaps significantly with the pyrite absorption. The resonant absorption between this source and a pyrite absorber is not greatly reduced from that for the pyrite absorber/pyrite source configuration, assuming equal probabilities of resonant emission from the two sources. Also, a minor improvement in resonant absorption can be obtained by vibrating the $^{57}$Co(Pd) source slightly. This is indicated in Figure 1, which shows the variation of transmission of radiation (count rate) through a pyrite-rich coal sample as a function of the vibration amplitude. The minimum in count rate is found to occur at a vibration amplitude of about 0.30 mm/s. Other standard Mossbauer sources, which incorporate $^{57}$Co in different non-magnetic metallic matrices (e.g. rhodium, copper, stainless steel, or chromium), could be used. However, the $^{57}$Co (Pd) source appears to overlap best with the pyrite absorption.

To a good approximation, it can be shown that

$$\frac{I_m}{I_s} = e^{kf_p m} \qquad (1)$$

where $I_m$ and $I_s$ are the intensities of the transmitted radiation when the source is in motion and at rest, respectively, $f_p$ is the mass fraction of pyrite in the coal sample of mass m per unit area, and k is a constant. Because of the highly specific nature of Mossbauer resonant absorption, this measurement would be unaffected by ash, moisture, organic sulfur, or any element other than iron.

The initial Mossbauer-based pyritemeter tested therefore consisted of a standard $^{57}$Co-doped palladium source in the usual Mossbauer geometry, with the output of the single-channel analyzer (used to isolate the 14.4 keV gamma-ray peak of interest) connected to a counting scaler.

Samples were prepared by adding measured weight percentages of -200 mesh pyrite (crushed from a single crystal) and quartz (crushed sea sand) to -60 mesh washed Pittsburgh seam coal. One-gram mixtures of the coal plus 0.5, 1.0, 1.5, 2.0 and 3.0 wt. percent pyrite and the coal plus 1.0, 2.0, 3.0, 5.0, and 8.0 wt. percent quartz were prepared. The coal was determined by Mossbauer spectroscopy to contain 1.12 wt. percent pyrite initially.

- 7 -

Count rates were measured on 500 mg aliquots of the mixtures and original coal in the same holder to facilitate comparison. Count rates were measured both with the source stationary ($I_s$) and with it strongly vibrating ($I_m$) for 18s intervals, in triplicate.

Data on the count rates, $I_s$ and $I_m$, are shown in Table I.

- 8 -

## TABLE I

Count-Rate Data* for Additions of Pyrite and Quartz
to Pittsburgh Coal

| Sample | $I_o^+$ | $I_s$ | $I_m$ | $I_m - I_s$ |
|---|---|---|---|---|
| Blank Holder | 683.3 | | | |
| Pittsburgh Coal | | 398.1 | 406.1 | 8.0 |
| Coal + 1/2% $FeS_2$ | | 384.6 | 394.9 | 10.3 |
| Coal + 1% $FeS_2$ | | 366.1 | 378.7 | 12.6 |
| Coal + 1-1/2% $FeS_2$ | | 351.3 | 365.3 | 14.0 |
| Coal + 2% $FeS_2$ | | 338.2 | 354.3 | 16.1 |
| Coal + 3% $FeS_2$ | | 306.7 | 325.0 | 18.3 |
| Coal + 1% $SiO_2$ | | 393.3 | 402.2 | 8.9 |
| Coal + 2% $SiO_2$ | | 389.4 | 397.7 | 8.3 |
| Coal + 3% $SiO_2$ | | 384.9 | 392.0 | 7.1 |
| Coal + 5% $SiO_2$ | | 378.0 | 385.8 | 7.8 |
| Coal + 8% $SiO_2$ | | 368.9 | 376.1 | 7.2 |

*Count rates expressed as 1000's per 18s, average
of three determinations. Standard deviation of
determination, approximately, $\pm 0.35$.

+Defined as the count rate through the blank holder.

- 9 -

The most readily apparent trend in the data is the decrease in count rate (increase in gamma-ray attenuation due to non-resonant absorption and scattering processes) with increasing additions of pyrite and quartz. These trends, unrelated to the Mossbauer resonant absorption process, indicate that the count rate is quite sensitive to the mineral matter or ash content of the coal. The difference $(I_m-I_s)$, increases with increasing pyrite but shows little change with the quartz content. This difference reflects the $^{57}$Fe Mossbauer resonant absorption process, which should be unaffected by quartz. These trends indicate that the count-rate measurements might be used to estimate not only pyrite but also ash in the coal.

The variation of count rate directly reflects the efficiency of gamma-ray attenuation by the coal sample. This attenuation is described by an exponential expression of the form:

$$\frac{I_m}{I_o} = e^{-um} \qquad (2)$$

where $I_m$ is the measured count rate of the gamma rays transmitted through the sample; $I_o$ is the count rate of the gamma rays incident on the sample; m is the mass of the sample per unit area exposed to the gamma rays; and u is the mass absorption coefficient for the sample.

- 10 -

For the constant-weight samples measured in this study, Equation (2) reduces to:

$$\ln(I_O/I_m) = a\,u \qquad (3)$$

and Equation (1) reduces to:

$$\ln(I_m/I_s) = a\,kf_p \qquad (4)$$

where a is a constant defined by the experimental conditions.

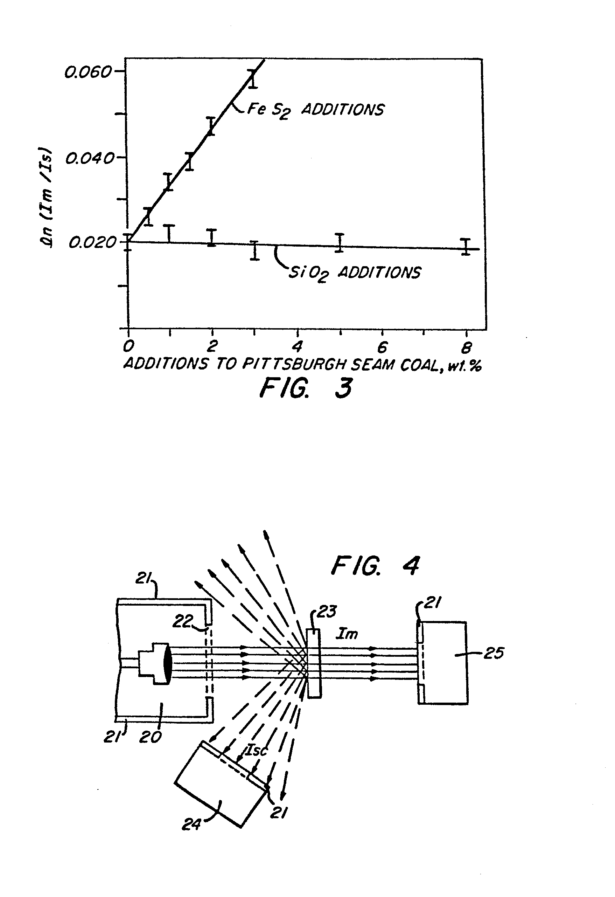

The raw data on count rates in Table I were therefore reduced to the logarithm of the ratios, $I_O/I_m$ and $I_m/I_s$, as listed in Table II. The variation of both of these parameters with the additions of pyrite is linear, as shown in Figures 2 and 3.

## TABLE II

Reduced Count-Rate Data for Additions of Pyrite and Quartz
to Pittsburgh Coal

| Sample | $\ln(I_m/I_s)$ | $\ln(I_o/I_m)$ |
|---|---|---|
| Pittsburgh Coal | 0.020 | 0.520 |
| Coal + 1/2% $FeS_2$ | 0.026 | 0.548 |
| Coal + 1% $FeS_2$ | 0.034 | 0.590 |
| Coal + 1-1/2% $FeS_2$ | 0.039 | 0.626 |
| Coal + 2% $FeS_2$ | 0.047 | 0.657 |
| Coal + 3% $FeS_2$ | 0.058 | 0.743 |
| | | |
| Coal + 1% $SiO_2$ | 0.022 | 0.530 |
| Coal + 2% $SiO_2$ | 0.021 | 0.541 |
| Coal + 3% $SiO_2$ | 0.018 | 0.556 |
| Coal + 5% $SiO_2$ | 0.020 | 0.572 |
| Coal + 8% $SiO_2$ | 0.019 | 0.597 |

- 12 -

The linear variation of $\ln(I_m/I_s)$ with pyrite content and lack of systematic variation with quartz shows that the Mossbauer-based pyritemeter concept is valid. However, the precision of a determination from this relationship is limited because of counting statistics. The standard deviation of $\ln(I_m/I_s)$ from this source of uncertainty is estimated to be $\pm 0.002$, which approximately corresponds to a $\pm 0.1$ wt.% uncertainty in pyritic sulfur.

The data shown in Figure 2 indicate that the variation in total count rate is also directly proportional to the addition of pyrite and is in fact more sensitive than $\ln(I_m/I_s)$. The standard error of an estimate of pyritic sulfur from $\ln(I_o/I_m)$ is $\pm 0.04$ wt.%. However, as shown in Figure 2, $\ln(I_o/I_m)$ is also sensitive to other minerals in coal, such as quartz. This is not the case for $\ln(I_m/I_s)$, which is specific for $^{57}Fe$ in pyrite and independent of all other elements in coal. Because the pyrite content normally varies considerably as a fraction of the mineral matter in coal, determinations of pyrite based on $\ln(I_m/I_s)$ should be much more broadly applicable.

These results indicated to us that the combination of $\ln(I_m/I_s)$ and $\ln(I_o/I_m)$ could be used to refine percent ash determinations from X-ray or

- 13 -

gamma-ray attenuation measurements because the pyrite, which is the major source of variation of $\ln(I_o/I_m)$, can be determined independently.

Figure 4 is a more or less diagrammatic illustration of the paths of gamma rays in the ash-monitoring of coal, wherein source 20 having a shield 21 emits gamma rays $I_o$ through aperture 22 to strike coal sample 23. The gamma rays $I_{sc}$ which are back-scattered are measured in detector 24, while those which are transmitted through coal sample 23, designated $I_m$ are measured in transmission detector 25. The three areas of the beams are known as the incident, transmitted, and back-scattered beams.

As shown in Figure 4, X-ray or gamma-ray attenuation can be measured in two ways: in transmission, with the sample between the source and the detector; or in reflectance, with the detector measuring the intensity, $I_{sc}$, of the back-scattered radiation. The transmission geometry is usually utilized in the Mossbauer experiment, whereas the back-scattered geometry is used in commercial X-ray ash-monitoring devices. However, there is a simple relationship between the two measurements:

$$I_{sc} = k(I_o - I_m) \qquad (5)$$

where k is a constant, principally defined by geometric factors. Consequently, the following description of

- 14 -

factors that affect the attenuation of Mossbauer gamma rays applies equally well to the back-scattered radiation utilized in ash-monitoring devices.

The critical factor to evaluate in Equation (2) is the mass-absorption coefficient (MAC), u, of the sample. Generally, the MAC is a function of the X-ray energy and the elemental composition of the sample. For a given X-ray energy, E,

$$u\ (E) = \sum_{i} u_i(e)\ f_i \qquad (6)$$

where $u_i(E)$ is the MAC for the $i^{th}$ element and $f_i$ is the mass fraction of the $i^{th}$ element in the sample.

It is the variation of the MAC with atomic number that forms the physical basis for the ash monitor. For X rays in the range 7 to 17 keV, there is a large difference between the MACs of the maceral-forming elements (H, C, N, O) and those of the ash-forming elements (Na, Mg, Al, Si, K, Ca, Fe). For the energy of the Mossbauer gamma rays (14.4 keV), the approximate MACs for selected elements and the major coal entities are shown in Table III.

- 15 -

## TABLE III

Approximate Mass-Absorption Coefficients for Selected
Elements and Coal Entities for X Rays of 14.4 KeV
(0.862 A Wavelength)

| Element | $\mu$ (14.4 keV) | Entity | $\mu$ (14.4 kev)*** |
|---------|------------------|--------|---------------------|
| H | 0.44 | Typical Maceral* | 0.9 + 0.17(%So) |
| C | 0.9 | | |
| N | 1.5 | Moisture, $(H_2O)$ | 1.9 |
| O | 2.1 | | |
| Na | 4.1 | Minerals: | |
| Mg | 7.8 | Quartz, $SiO_2$ | 7 |
| Al | 10.3 | Kaolinite, $Al_2Si_2O_5(OH)_4$ | 6 |
| Si | 12.5 | Illite, K-Al-Si-O,OH** | 7.5 - 8.0 |
| S | 18 | Calcite, $CaCO_3$ | 14 |
| Cl | 21 | Chlorite, $Fe_5Al_2Si_3O_{10}(OH)_8$ | 28 |
| K | 28 | Siderite, $FeCO_3$ | 32 |
| Ca | 32 | Pyrite, $FeS_2$ | 39 |
| Fe | 64 | | |

\* Typical maceral C:H:N:O = 89:6:2:3, %So = % organic sulfur

\*\* Variable: $KAl_5SI_7O_{20}(OH)_4$ to $K_2Al_6Si_6O_{20}(OH)_4$

\*\*\* Units are $cm^2/g$.

Data estimated from "X-ray Absorption and Emission in Anayltical Chemistry" by H. A. Liebhafsky, H. G. Pfeiffer, E. H. Winslow and P. D. Zemany, J. Wiley and Sons, New York, 1960.

- 16 -

As indicated in Table III, the mineral matter may attenuate such radiation between 4 and 40 times more efficiently than the macerals, depending on the actual mineralogy of the coal and the sulfur content of the macerals. Thus, it is a gross simplification to make the assumption, which is the basis for ash-monitoring devices, that u is derived from just two components, $u_m$ and $u_a$, the MACs for the macerals and the mineral matter (ash), respectively:

$$u = (1 - f_a) u_m + f_a u_a \qquad (7)$$

where $f_a$ is the mass fraction of the ash. However, although this equation is inadequate in the general case, it does have merit in those situations where $u_m$ and $u_a$ can be considered constant. As a result, it is generally appreciated that X-ray ash-monitoring devices are limited to such applications as monitoring the coal from a single mine or a constant blend of coals from a preparation plant where the product is reasonably uniform for a long period of time.

We have extended this concept somewhat further. Examination of Table III indicates that the iron-bearing minerals give rise to much of the variation of $u_a$ with mineralogy. Furthermore, it is seen that sulfur has by far the largest MAC of the

- 17 -

elements commonly present in organic combination in coal macerals. These observations suggest that Equation (7) should be replaced by an expression of the form

$$u = (1 - f_a)u'_m + (f_a - f_{Fe})u_{(a-Fe)} +$$
$$f_{Fe}u_{Fe} +$$
$$f_S u_S \qquad\qquad (8)$$

where $u_{Fe}$, $u_S$ are the MACs for the elements iron and sulfur, $u_{(a - Fe)}$ is the MAC for the iron-free ash, $u'_m$ is the MAC for the sulfur-free maceral component, and $f_{Fe}$ and $f_S$ are the mass fractions of iron and sulfur in the coal, respectively.

This equation would appear to have a distinct advantage over Equation (7) for the general case, as the four MACs involved should be more or less constant for any coal. This equation, as well as Equation (7) above, was experimentally tested, as discussed below.

The experimental work consisted of measuring the transmission of radiation (count rate) from a $^{57}$Co(Pd) source through 20 coal samples. As before, the same holder and same weight of -60 mesh coal (500 mg) were used for all samples to minimize experimental variation and facilitate comparison. The count rate was measured with the $^{57}$Co(Pd) source vibrating with a small amplitude (0.4 mm/s) and a large amplitude (12

- 18 -

mm/s). Each count rate was determined for 18-second intervals, in triplicate, and the count rates recorded in Table IV are averages of the three determinations. The count rate for the empty holder in the gamma-ray beam was used as a measure of $I_o$, the intensity of the gamma rays incident on the sample.

The 20 coals chosen were ones for which proximate and sulfur analyses and Mossbauer data were available. These coals exhibit a wide range in all the parameters of interest: ash contents from 4.0 to 34.0 wt.%, sulfur contents from 0.31 to 3.92 wt.%, iron contents from 0.07 to 1.78 wt.%. The pertinent data from these analyses and the parameters $\ln(I_o/I_m)$ and $\ln(I_m/I_s)$ derived from Table IV, are listed in Table V for the 20 coals. Most coals were available in both raw and washed (lower ash, lower pyrite) forms; these are designated by R and W, respectively, in Tables IV and V.

## TABLE IV

### Count Rates* Measured for Various Coals

| Coal | $I_o$ | $I_m$ | $I_s$ |
|---|---|---|---|
| **1st Group** | 661.2 | | |
| Gurnee, W | | 439.2 | 431.0 |
| Gurnee, R | | 238.7 | 225.9 |
| Crystal Block, W | | 448.1 | 441.9 |
| Crystal Block, R | | 310.1 | 299.9 |
| Somerset D, W | | 366.1 | 365.1 |
| Somerset D, R | | 379.2 | 376.0 |
| Maple Creek, W | | 401.4 | 389.2 |
| **2nd Group** | 727.4 | | |
| Maple Creek, R | | 288.5 | 276.7 |
| Cumberland, R | | 366.5 | 348.3 |
| Cumberland, W | | 431.5 | 419.9 |
| Dilworth, R | | 305.9 | 291.1 |
| Dilworth, W | | 424.4 | 415.4 |
| Illinois #6 | | 308.4 | 288.4 |
| **3rd Group** | 702.0 | | |
| Pocahontas #3, R | | 323.6 | 314.3 |
| Pocahontas #3, W | | 401.1 | 376.2 |
| Somerset E, W | | 420.4 | 414.5 |
| Somerset E, R | | 435.5 | 431.1 |
| Lynch, R | | 317.2 | 306.5 |
| Somerset C, R | | 414.8 | 411.1 |
| Concord, W | | 442.1 | 436.4 |

* Expressed as 1000's per 18 s -

average of three determinations.

## TABLE V

### Pertinent Analytical and Reduced Count-Rate Data for Twenty Coals

| Coal | $\ln(I_O/I_m)$ | $\ln(I_m/I_B)$ |
|---|---|---|
| Gurnee, W | 0.409 | 0.019 |
| Gurnee, R | 1.019 | 0.055 |
| Crystal Block, W | 0.389 | 0.014 |
| Crystal Block, R | 0.757 | 0.033 |
| Somerset D, W | 0.591 | 0.003 |
| Somerset D, R | 0.556 | 0.008 |
| Maple Creek, W | 0.499 | 0.031 |
| Maple Creek, R | 0.925 | 0.042 |
| Cumberland, R | 0.685 | 0.052 |
| Cumberland, W | 0.523 | 0.026 |
| Dilworth, R | 0.866 | 0.050 |
| Dilworth, W | 0.539 | 0.021 |
| Illinois #6 | 0.857 | 0.068 |
| Pocahontas #3, R | 0.774 | 0.029 |
| Pocahontas #3, W | 0.560 | 0.012 |
| Somerset E, R | 0.513 | 0.014 |
| Somerset E, W | 0.478 | 0.009 |
| Lynch, R | 0.794 | 0.034 |
| Somerset C, R | 0.526 | 0.009 |
| Concord, W | 0.462 | 0.013 |

## TABLE V (CONTINUED)

| | Wt% in Coal | | | |
| %Ash | %S$_{pyr}$[+] | %S$_{coal}$ | %Fe$_{coal}$ | %Fe$_{pyr}$[+*] |
|---|---|---|---|---|
| 4.9 | 0.29 | 0.78 | 0.58 | 44 |
| 34.0 | 0.57 | 0.90 | 1.78 | 28 |
| 5.8 | 0.22 | 0.86 | 0.33 | 59 |
| 24.5 | 0.43 | 0.93 | 0.99 | 38 |
| 4.0 | 0.01 | 0.32 | 0.07 | 12 |
| 5.4 | 0.02 | 0.31 | 0.12 | 15 |
| 10.7 | 0.62 | 1.43 | 0.63 | 86 |
| 30.4 | 0.84 | 1.30 | 0.99 | 74 |
| 9.4 | 1.51 | 2.82 | 1.32 | 100 |
| 6.4 | 0.60 | 2.47 | 0.56 | 94 |
| 23.1 | 1.19 | 1.65 | 1.18 | 88 |
| 9.7 | 0.45 | 1.26 | 0.46 | 85 |
| 12.5 | 1.79 | 3.92 | 1.61 | 97 |
| 22.5 | 0.16 | 0.58 | 0.93 | 15 |
| 5.8 | 0.07 | 0.53 | 0.32 | 19 |
| 8.8 | 0.12 | 0.62 | 0.48 | 22 |
| 4.3 | 0.06 | 0.69 | 0.35 | 15 |
| 24.9 | 0.55 | 1.36 | 0.91 | 53 |
| 11.9 | 0.11 | 0.62 | 0.31 | 31 |
| 7.6 | 0.21 | 0.78 | 0.41 | 45 |

* Percentage of iron in the form of pyrite.
+ Derived from Mössbauer data.

- 22 -

To evaluate the Mossbauer pyritemeter and ash monitor, the data in Table V were subjected to statistical analysis.  Routines used in this work were correlation and multiple linear regression.

The correlation matrix for the data in Table V is shown in Table VI.

— 23 —

TABLE VI

Correlation Coefficient (r) Matrix for Data in TABLE V

| Variable | Variable | | | | | | |
|---|---|---|---|---|---|---|---|
| | 1 | 2 | 3 | 4 | 5 | 6 | 7 |
| 1. $\ln(I_o/I_m)$ | 1.0 | | | | | | |
| 2. $\ln(I_m/I_s)$ | 0.788 | 1.0 | | | | | |
| 3. %Ash | 0.880 | 0.632 | 1.0 | | | | |
| 4. %$S_{pyr}$ | 0.552 | 0.901 | 0.312 | 1.0 | | | |
| 5. %$S_{coal}$ | 0.330 | 0.759 | 0.071 | 0.916 | 1.0 | | |
| 6. %$Fe_{coal}$ | 0.825 | 0.963 | 0.761 | 0.778 | 0.618 | 1.0 | |
| 7. %$Fe_{pyr}$* | 0.170 | 0.627 | 0.086 | 0.814 | 0.831 | 0.440 | 1.0 |

\* Percentage of iron in the form of pyrite.

- 24 -

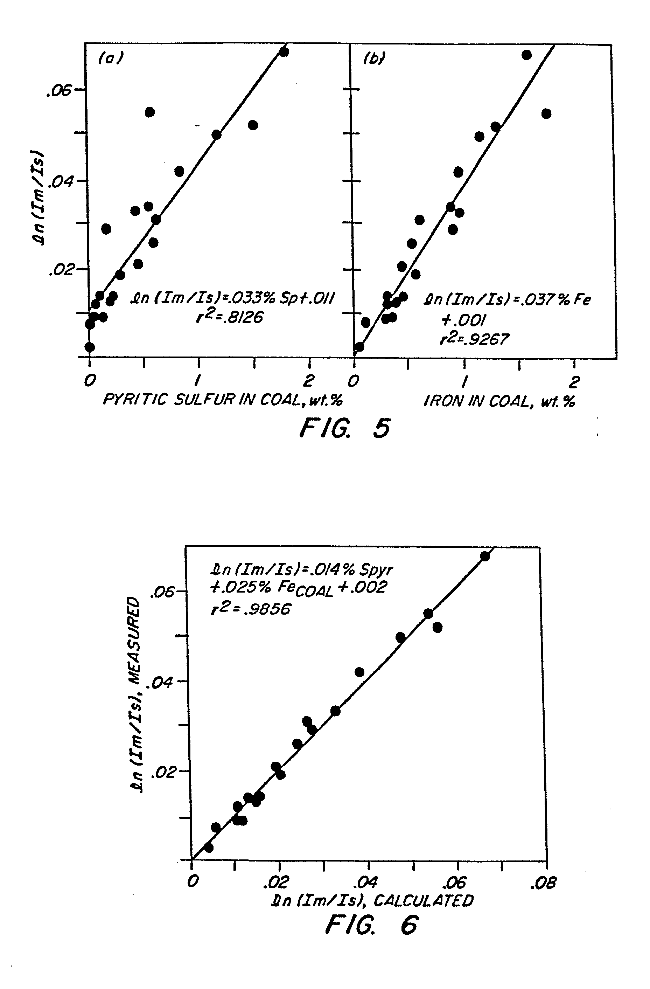

For the pyritemeter, the best correlation with $\ln(I_m/I_s)$ was shown by $\%Fe_{coal}$, rather than $\%S_{pyr}$. Plots of these parameters against $\ln(I_m/I_s)$ are shown in Figure 5. The derived equations from the regression analysis are as follows:

$$\ln(I_m/I_s) = 0.033 \; \%S_{pyr} + 0.011 \qquad (9)$$

for which $r^2 = 0.8126$ and the standard error of an estimate of

$\ln(I_m/I_s)$ is $\pm 0.008$,

and

$$\ln(I_m/I_s) = 0.037 \; \%Fe_{coal} + 0.001 \qquad (10)$$

for which $r^2 = 0.9267$ and the standard error of an estimate of

$\ln(I_m/I_s)$ is $\pm 0.005$. Obviously, from Figure 5 and the regression-analysis parameters, Equation (10) is a better explanation for the variation of $\ln(I_m/I_s)$ than Equation (9). This implies that $\ln(I_m/I_s)$ is more closely related to $\%Fe_{coal}$ than to $\%S_{pyr}$.

An improvement over both these equations is the equation derived by multiple regression involving both parameters:

$$\ln(I_m/I_s) = 0.025 \; \%Fe_{coal} + 0.014 \; \%S_{pyr} + 0.002 \qquad (11)$$ for which $r^2 = 0.9856$ and the standard error of an estimate of $\ln(I_m/I_s)$ is 0.002. A plot

of measured $\ln(I_m/I_s)$ against $\ln(I_m/I_s)$ calculated according to Equation (11) is shown in Figure 6.

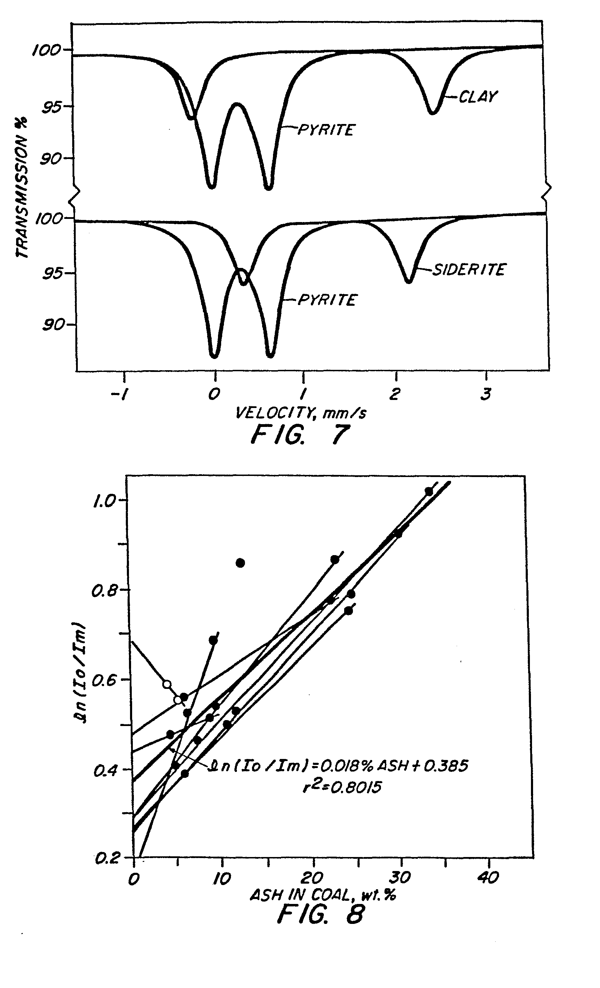

These findings show that the Mossbauer pyritemeter is sensitive not only to iron in pyrite but also to iron in other mineral forms in the coal. This is not too surprising when we consider that both siderite ($FeCO_3$) and iron-bearing clays, the two principal iron-bearing minerals in coal other than pyrite, have Mossbauer absorption peaks that overlap significantly with the pyrite Mossbauer absorption peaks, Figure 7. These other iron minerals can comprise a major source of iron in coal and would therefore contribute to Mossbauer resonant absorption with the $^{57}Co(Pd)$ source at rest or vibrating at a small velocity amplitude.

These results suggest that the Mossbauer pyritemeter will not be adequate for measurements of pyrite in random coals, as might be encountered in a research laboratory. However, it would be extremely useful under certain restricted conditions, such as monitoring pyrite in coals in which pyrite is known to be the principal iron-bearing mineral. Nearly all U. S. lignites and subbituminous coals, as well as most moderate- to high-sulfur bituminous coals do in fact contain iron primarily ( 75%) in the form of pyrite.

As removal of pyrite is one of the main aims of coal-cleaning operations, particularly for steam-coal use, such a pyritemeter could monitor pyrite removal.

As the measurement of $\ln(I_m/I_s)$ reflects not only pyrite but also other forms of iron in the coal, it can also be used as a measure of the iron content of the coal. In a subsequent discussion, this measurement combined with measurements of $\ln(I_o/I_m)$ is shown to lead to more precise determinations of the percent ash in coal.

The regression analysis for the ash monitor is based on a reduced data set of 18 coals. The Somerset D coals were omitted from the analysis because of their unusual halogen contents. Chemical analyses of these coals indicate that they contain about 0.5 wt.% of both chlorine and bromine. The latter element is extremely efficient for absorption of 14.4 keV gamma rays.

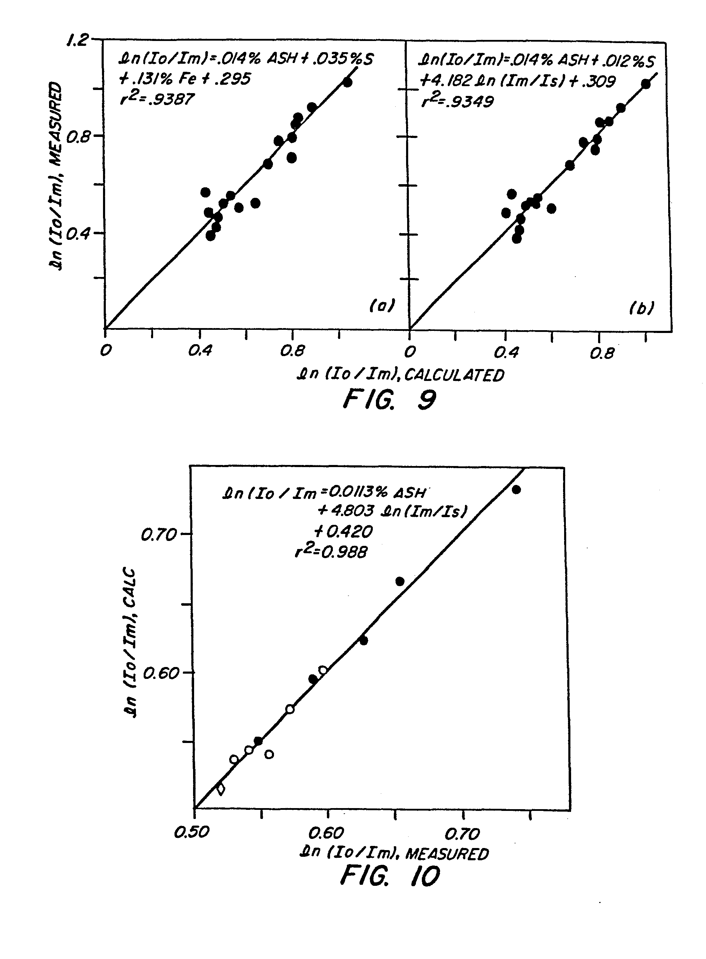

The variable, %Ash, exhibits the best correlation with $\ln(I_o/I_m)$. However, as indicated in Figure 8, this variable is by no means sufficient to account for the variation in $\ln(I_o/I_m)$. The derived regression equation

$$\ln(I_o/I_m) = 0.018 \text{ %Ash} + 0.384 \qquad (12)$$

has a correlation coefficient of $r^2 = 0.8015$ and the standard error of an estimate of $\ln(I_o/I_m)$ is

- 27 -

0.089. This equation, of course, corresponds to a rearranged form of Equations (2) and (7):

$$\underline{\ln(I_o/I_m)} = u = f_a(u_a - u_m) + u_m$$
$$m$$

(13)

where %Ash $= 100f_a$.

Therefore this equation, which is the basis for commercial ash-monitoring devices, is obviously inadequate for the general case. Note, in particular, the variation in $u_m$ implied by the different intercepts on the y axis in Figure 8 for lines connecting data points for the same coals in raw and washed form.

As earlier noted Equation (8) should be more applicable to the general case. This equation, rearranged, is as follows:

$$\underline{\ln(I_o/I_m)} = u = u'_m + f_a[u_{(a - Fe)} -$$
$$m$$
$$u'_m]$$

$$+ f_{Fe}[u_{Fe} - u_{(a - Fe)}] +$$

$$f_s u_s \qquad (14)$$

This equation, with %Ash ($= 100 f_a$), %Fe ($= 100 f_{Fe}$) and %S ($= 100 f_s$) used as variables, was tested by multiple linear regression. The best-fitting equation is as follows:

- 28 -

$$\ln(I_o/I_m) = 0.014 \text{ \%Ash} + 0.035 \text{ \%S}$$
$$+ 0.131 \text{ \%Fe} + 0.295 \tag{15}$$

for which $r^2 = 0.9387$ and the standard error of an estimate of $\ln(I_o/I_m)$ is 0.053. The relationship between $\ln(I_o/I_m)$ measured and $\ln(I_o/I_m)$ calculated according to Equation (15) is shown in Figure 9a.

The variables $\text{\%Fe}_{coal}$ and $\ln(I_m/I_s)$ were found to be strongly correlated, as discussed above for the pyritemeter, and an alternative version of Equation (15) was therefore also derived by multiple linear regression:

$$\ln(I_o/I_m) = 0.014 \text{ \%Ash} + 0.012 \text{ \%S} +$$
$$4.182 \ln(I_m/I_s) + 0.309$$

$$(16)$$

for which $r^2 = 0.9349$ and the standard error of an estimate of

$\ln(I_o/I_m)$ is $\pm 0.054$. This equation is very similar to

Equation (15) above, as shown in Figure 9b.

One important difference, however, is the much smaller coefficient in Equation (16) for %S. This variable also had a large standard error associated with the coefficient ($\pm 0.365$) and a Student's t value

- 29 -

(a test of the significance of the variable) much lower than for the other two variables.  These factors suggested that the variable, %S, was not important in Equation (16).  It was therefore dropped from the regression analysis and the following equation was derived:

$$\ln(I_o/I_m) = 0.013 \text{ %Ash} +$$
$$4.884 \ln(I_m/I_s) + 0.316 \qquad (17)$$

for which $r^2 = 0.9344$ and the standard error of an estimate of $\ln(I_o/I)$ is $\pm 0.053$.

The three equations, (15) through (17), all offer approximately similar explanations for the variation of $\ln(I_o/I_m)$.  However, Equation (17) is especially intriguing because it can be rearranged as follows:

$$\text{%Ash} = 77.9 \ln(I_o/I_m) - 380.5 \ln(I_m/I_s) +$$
$$24.6 \qquad (18)$$

and suggests that %Ash can be determined from measurement of $\ln(I_o/I_m)$ and $\ln(I_m/I_s)$.  As $\ln(I_m/I_s)$ reflects variation in iron content and also, to a lesser extent, in sulfur content (as $FeS_2$), the principal sources of variability and uncertainty of the simple ash monitor in the general case are therefore taken into account.  However, the standard error of an estimate of %Ash with Equation (18) exceeds ±4 wt.%, which is still unsatisfactory for many purposes and suggests that refinements such as we have considered here are in many cases not sufficient for general determinations of ash content.

For the specific case, however, Equation (18) is a considerable improvement over existing X-ray ash-monitor technology as it should enable more precise estimates of ash content to be made and allow more latitude in the types of problems that might be addressed by ash-monitoring devices. For example, regression analysis of the data presented in Tables I and II for additions of pyrite and quartz to a specific sample of Pittsburgh seam coal yields the following equation:

$$\ln(I_o/I_m) = 0.0113 \text{ %Ash} +$$
$$4.803 \ln(I_m/I_s) + 0.420 \qquad (19)$$

for which $r^2 = 0.9878$ and the standard error of an estimate of $\ln(I_o/I)$ is 0.008. This error is equivalent to an error of ±0.7 wt.% in %Ash and arises mostly from the large relative error in $\ln(I_m/I_s)$. A plot of $\ln(I_o/I_m)$ measured against $\ln(I_o/I)$ calculated according to Equation (19) is shown in Figure 10. Thus, the effect of such disparate minerals as quartz

- 31 -

and pyrite on $\ln(I_o/I_m)$ can be fully accounted for by inclusion of $\ln(I_m/I_s)$. It is therefore proposed that simple coal blends of varying proportions may be adequately monitored by a Mossbauer-based device that measures both $\ln(I_o/I_m)$ and $\ln(I_m/I_s)$.

One further observation should be noted regarding the use of $^{57}$Co Mossbauer sources in connection with coal parameter measurements. When $^{57}$Co decays, it emits gamma rays of energy, 123 keV, in addition to the 14 keV Mossbauer gamma rays that have been the focus of this work up to now. The mass-absorption coefficients for all common elements in coal (H, C, N, O, Al, Si, S, Ca, Fe, etc.) are quite similar at 123 keV. As a result, the attenuation of gamma rays of this energy can be used to monitor the weight or thickness of coal in the gamma-ray beam.

To demonstrate this point, an experiment was conducted in the transmission geometry (Figure 4), with a sodium iodide detector replacing the proportional counter because of its higher sensitivity to gamma rays of 123 keV. The attenuation of these gamma rays was then measured for raw and washed samples of two coals of different pyrite and ash contents. The attenuation parameter, $\ln(I_o^{123}/I^{123})$, where $I_o^{123}$ is the intensity of the 123 keV gamma rays incident on the sample and $I^{123}$ is the intensity of the transmitted

- 32 -

beam, was measured, in a manner similar to that described above for the 14 keV gamma rays, by recording the count rates for different weights of coal and the empty sample holder.  As can be seen from the experimental data shown in Table VII, the four samples gave closely similar values for the attenuation of 123 keV gamma rays for the same weight of coal, despite the large ranges in ash content (6.7 to 31.5 wt.%) and pyritic sulfur (0.23 to 1.46 wt.%).

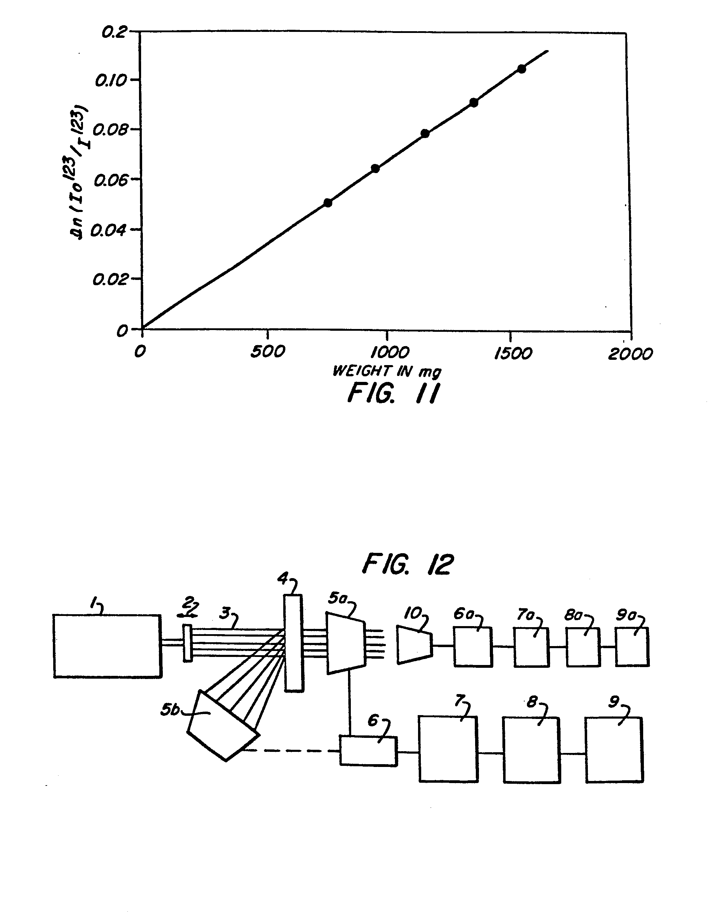

This lack of variation demonstrates that the attenuation of 123 keV gamma rays from the $^{57}$Co source is independent of the ash and pyrite contents. In Figure 11, the averages of the determinations for the four coals are plotted against weight of coal in the gamma-ray beam and an excellent linear correlation is found.  Thus, the attenuation of the 123 keV gamma rays may be used as a measure of the weight or thickness of coal in the gamma-ray beam.  This measurement, in connection with the 14 keV gamma-ray measurements, may be used to determine the weight of coal samples taken by an automated sampling device, thereby avoiding weighing of the sample in an automated method of determining ash and pyrite contents of coal.

## TABLE VII

### Count-rate Data* for Attenuation of 123 KeV Gamma rays For Four Coal Samples

| Weight, mg | Raw Coal | | |
|---|---|---|---|
| | $I_o^{123}$ | $I^{123}$ | $\ln(I_o^{123}/I^{123})$ |
| **Coal A** | | | |
| 0, (blank) | 2059 | | |
| 750 | | 1955 | 0.052 |
| 950 | | 1928 | 0.066 |
| 1150 | | 1903 | 0.079 |
| 1350 | | 1878 | 0.092 |
| 1550 | | 1851 | 0.106 |
| **Coal B** | | | |
| 0, (blank) | 2052 | | |
| 750 | | 1948 | 0.052 |
| 950 | | 1923 | 0.065 |
| 1150 | | 1895 | 0.080 |
| 1350 | | 1873 | 0.091 |
| 1550 | | 1846 | 0.106 |
| Standard Error: | ±0.7 | ±0.7 | ±0.0015 |

TABLE VIII  (CONTINUED)

| | Washed Coal | | |
|---|---|---|---|
| | $I_o^{123}$ | $I^{123}$ | $\ln(I_o^{123}/I^{123})$ |
| | 2058 | | |
| | | 1958 | 0.050 |
| | | 1929 | 0.065 |
| | | 1905 | 0.077 |
| | | 1881 | 0.090 |
| | | 1857 | 0.103 |
| | 2057 | | |
| | | 1956 | 0.050 |
| | | 1928 | 0.065 |
| | | 1903 | 0.078 |
| | | 1878 | 0.091 |
| | | 1850 | 0.106 |
| Standard Error: | ±0.7 | ±0.7 | ±0.0015 |

*Count rates expressed as 1000's per 30s,
average of four determinations

| | Wt.% Ash | Wt.% Pyritic Sulfur |
|---|---|---|
| Coal A raw: | 31.47 | 0.47 |
| Coal A washed: | 6.70 | 0.23 |
| Coal B raw: | 15.88 | 1.46 |
| Coal B washed: | 7.20 | 0.68 |

- 35 -

A preferred configuration of our combined ash meter and pyrite (iron) meter is shown more or less diagrammatically in Figure 12.

In Figure 12, conventional shielding devices and circuitry are omitted. Determinations are made by measuring the 14 KeV gamma-ray count rates with a proportional counter or other appropriate detector, 5a or 5b, connected to an electronic counting device 9 by a preamplifier 6, amplifier 7 and single-channel or region-of-interest device 8. For each sample 4, two count rates are measured; one with the source 2 at rest or vibrating very slightly (achieving a maximum speed of 1 mm/sec); the other with the source vibrating strongly (wherein the greatest speed achieved is at least 5 mm/sec, preferably at least 10 mm/sec) to destroy the Mossbauer resonant absorption process. The difference in count rate between the two situations is proportional to the weight percent of iron in the coal sample, whereas the total count rate is related to both percent ash and percent iron. By means of a calibration equation based on known characteristics of the particular coal source, % ash, % Fe, and % pyritic sulfur can be determined from the measurements. The measurement may be performed in either transmission or scattering geometry. These are indicated by alternative positionings of the detector, 5a and 5b,

- 36 -

relative to the sample 4, and source oscillator 1.

Optionally, to estimate the weight or thickness of coal in the gamma-ray beam, a detector 10 can be placed behind the first detector 5a, to measure 123 keV gamma-ray count rates. To measure this third count rate, connection is required from the detector to an electronic counting device, 9A, by a preamplifier 6A, amplifier 7A, and a single-channel or region-of-interest device 8A. If a suitably sensitive detector is available, measurement of both the 14 keV and 123 keV gamma ray count rates may be incorporated into a single detector/recording system.

The variation of the iron content of coal is usually the main source of error in percent-ash determinations based solely on gamma- or X-ray absorption and scattering measurements. Thus, the combination of both measurements in the single device described herein results in a significant improvement in the precision of determinations of percent ash in coal. For most coals, pyrite is the major iron-bearing mineral and the determination of the Mossbauer resonant absorption provides a rapid, direct measurement of the weight percent of pyrite or pyritic sulfur in coal. Both measurements, percent ash and percent pyritic sulfur, are of considerable value in many coal-industry applications. The measurements are considerably more

- 37 -

rapid (on the order of a few minutes) than standard ASTM methods for determining pyritic sulfur and ash. By use of automatic sampling techniques and the possible incorporation of means to detect the 123 keV gamma radiation, the device may be adapted for use in on-line monitoring situations.

The most practical Mossbauer-based devices for the rapid measurement of pyritic sulfur and ash contents of coal appear to be restricted to applications involving a specific coal (from a source known to have compositions within certain ranges) or suite of coals. This is because the pyritemeter reflects other forms of iron in the coal in addition to pyrite and because the absorption of $^{57}Fe$ 14 keV Mossbauer gamma rays by coal is a function not only of the ash content, but also the sulfur (and chlorine) contents of the macerals, the ratio of iron-bearing minerals (pyrite, siderite, chlorite) to iron-free minerals (quartz, illite, kaolinite, etc.), and the moisture content of the coal. Therefore, application of these techniques will require that each individual coal source be separately calibrated.

Such Mossbauer devices could be extremely useful for specific applications, such as monitoring the pyrite and ash content of coals in which pyrite is the principal iron-bearing mineral. Additionally, combining the attenuation and pyritemeter measurements in a single device provides a more precise determination of the ash content of a coal than is possible from X-ray absorption measurements alone.

These combined measurements could also enable a broader range of applications than is currently possible to be addressed by ash-monitoring devices. Such measurements could be very useful in preparation or coke-plant laboratories.

0113194

- 39 -

CLAIMS:-

1. A method of determining iron, pyrite or ash content of coal, comprising measuring the attenuation or scattering by a coal sample of 14 kV gamma rays from a Mossbauer source of $^{57}$Co in a non-magnetic metallic matrix, characterized in that the measuring is performed both with the source at rest or vibrating with a peak speed of less than 1mm/sec and also with the source vibrating with a peak speed of at least 5mm/sec and generating respective output signals representing said attenuation or scattering.

2. A method as claimed in claim 1, characterized in that the second-mentioned measurement is conducted with the source vibrating with a peak speed of at least 10mm/sec.

3. A method as claimed in claim 1 or claim 2, characterized in that the Mossbauer source comprises $^{57}$Co-doped palladium.

4. A method as claimed in any one of claims 1 to 3, characterized by generating an output signal which represents the ratio of the two measurements.

5. A method as claimed in claim 4, characterized by determining the pyrite and iron contents of said sample from:

$$\text{wt.\% of pyrite in coal} = A \ln\left(\frac{I_m}{I_s}\right) + B$$

- 40 -

$$\text{wt.\% of iron in coal} = C \ln \left( \frac{I_m}{I_s} \right) + D$$

where $I_s$ and $I_m$ are the first and second-mentioned measurements, respectively, and A, B, C, and D are predetermined coefficients that are dependent on the concentrations of iron in different forms in representative coal samples from the same coal source.

6. A method as claimed in claim 5, characterized in that the two equations are replaced by:

$$\text{wt.\% of pyrite} = A \quad \frac{I_m - I_s}{I_s} + B$$

and,

$$\text{wt.\% of iron} = C \quad \frac{I_m - I_s}{I_s} + D$$

7. A method as claimed in any one of claims 1 to 4 for determining ash content, characterized by generating an output signal $I_o$ representative of the attenuation or scattering from said source with no sample present and determining the ash content from:

$$\text{wt.\% of ash in coal} = F \ln \left( \frac{I_o}{I_m} \right) + G \ln \left( \frac{I_m}{I_s} \right) + H$$

where $I_s$ and $I_m$ are the first and second-mentioned measurements and F, G, and H are coefficients determined by the mineral and maceral constitution of the coal and have been predetermined for samples of known ash, mineral, maceral and iron contents.

8. A method for determining ash content of coal, characterized by measuring the weight percentage of iron or pyrite in a coal sample by the method of claim 1, measuring the attenuation or scattering by said sample of gamma rays or X rays from any other source, and using said measurements in experimentally derived formulae that relate the ash content of the coal to both the attenuation or scattering of said gamma rays or X rays and the iron or pyrite content of the coal.

9. A method as claimed in any preceding claim, characterized by determining the weight or thickness of the coal sample by measuring the attenuation by said coal sample of the 123 keV gamma rays emitted by the Mossbauer source.

FIG. 1

FIG. 2

FIG. 3

FIG. 4

(a)

$\ln (Im/Is)$

.06

.04

.02

$\ln (Im/Is)=.033\% Sp+.011$
$r^2=.8126$

0     1     2

PYRITIC SULFUR IN COAL, wt.%

(b)

$\ln (Im/Is)=.037\% Fe$
$+.001$
$r^2=.9267$

0     1     2

IRON IN COAL, wt.%

FIG. 5

$\ln (Im/Is), MEASURED$

$\ln (Im/Is)=.014\% Spyr$
$+.025\% Fe_{COAL}+.002$
$r^2=.9856$

.06

.04

.02

0     .02     .04     .06     .08

$\ln (Im/Is), CALCULATED$

FIG. 6

FIG. 7

$\ln(I_o/I_m) = 0.018\% \ ASH + 0.385$

$r^2 = 0.8015$

FIG. 8

516

0113194

FIG. 9

$\mathcal{L}n(Io/Im)=.014\% ASH+.035\%S+.131\% Fe+.295$
$r^2=.9387$

$\mathcal{L}n(Io/Im)=.014\% ASH+.012\%S+4.182 \mathcal{L}n(Im/Is)+.309$
$r^2=.9349$

$(a)$

$(b)$

$\mathcal{L}n(Io/Im)$, MEASURED

$\mathcal{L}n(Io/Im)$, CALCULATED

FIG. 10

$\mathcal{L}n(Io/Im=0.0113\% ASH+4.803 \mathcal{L}n(Im/Is)+0.420$
$r^2=0.988$

$\mathcal{L}n(Io/Im)$, CALC

$\mathcal{L}n(Io/Im)$, MEASURED

0113194

FIG. 11

FIG. 12